# EUROPÄISCHE PATENTSCHRIFT

(11) **EP 0 537 551 B1**
(45) Veröffentlichungstag und Bekanntmachung des Hinweises auf die Patenterteilung: **02.05.1997**
(21) Anmeldenummer: 92116710.2
(22) Anmeldetag: 30.09.1992
(51) Int. Cl.: C12M 3/04, C12M 1/04

(54) **Drucksteuersystem und Druckregelsystem für eine Vorrichtung zur Zellkultivierung und Gewinnung von Zellprodukten**
Pressure control system for an apparatus for cell cultivation and cell-product harvesting
Système de contrôle de pression d'un dispositif pour la culture de cellules et la récupération des produits

(30) Priorität: 18.10.1991 US 779082
(43) Veröffentlichungstag der Anmeldung: 21.04.1993
(73) Patentinhaber: Integra Biosciences GmbH, 35463 Fernwald (DE); ENDOTRONICS INC., Minneapolis, MN 55433 (US)
(72) Erfinder: Wilson, John R., c/o Endotronics Inc., Minneapolis, MN 55433 (US); Kowol, Ewald c/o Tecnomara Deutschland GmbH, W-6301 Fernwald (DE)
(74) Vertreter: Linser, Heinz

(56) Entgegenhaltungen:
- EP-A- 0 334 506
- WO-A-90/10690
- WO-A-90/13639
- US-A- 4 647 539

## Beschreibung

Die Erfindung betrifft ein Drucksteuersystem und Druckregelsystem für eine Vorrichtung zur Zellkultivierung und Gewinnung von Zellprodukten, im folgenden Bioreaktor genannt, mit einem Gasraum und einen darin befindlichen Zellkulturraum sowie Einrichtungen zur Gaszufuhr und Gasabfuhr, die mit dem Bioreaktor verbunden sind, ferner Einrichtungen zur Zufuhr von flüssigen Nährstoffen und zur Entfernung flüssiger Abfallprodukte, welche getrennt von den Einrichtungen zur Gaszufuhr und Gasabfuhr an dem Bioreaktor angeschlossen sind.

Die "in-vitro-Kultur" von lebenden Zellen wird für eine Vielfalt von Anwendungen einschließlich der Herstellung viraler Vakzine, sowie der Gewinnung wertvoller Nebenprodukte des Zellmetabolismus und der Produktion gewebeähnlicher Derivate zur Herstellung künstlicher Organe eingesetzt.

Es ist bereits ein gewerbsmäßig angewendetes Verfahren zur Züchtung lebender Zellen in-vitro bekannt, welches die sogenannte Hohlfasertechnologie verwendet. Die Hohlfasertechnologie bietet einige Vorteile, wie beispielsweise eine hohe Stoffkonzentration sowie eine hohe Reinheit und Zelldichte. Ein Haupthindernis bei der wirksamen Nutzung des Zellraums besteht bei den bekannten in-vitro Bioreaktoren in der Sauerstoffversorgung der Zellen.

Um bei den bekannten Hohlfasermethoden Zellen mit Sauerstoff zu versorgen, wird ein flüssiges Medium vor seiner Zufuhr zur interkapillaren Seite der Hohlfaser mit Sauerstoff angereichert. Der Sauerstoff wird dabei mittels Diffusion durch die Faserwand zur Umgebung der Zellen übertragen. Diese Versorgungsmethode weist jedoch den Nachteil auf, daß ein wäßriges Medium, welches sich mit Luft im Gleichgewichtszustand befindet, unter Normalbedingungen (37°C,[760 mm Hg] 1013,2 mbar) nur 0.2 mMol Sauerstoff pro Liter aufnehmen kann. Aus diesen Gründen muß dem Bioreaktor ein großes Volumen des Mediums zugeführt werden, um eine relativ kleine Menge Sauerstoff zur Verfügung stellen zu können.

Mit steigendem Sauerstoffbedarf einer wachsenden Zellkultur steigen auch die Zuführungsraten des mit Sauerstoff angereicherten Mittels. Hieraus ergeben sich mechanische und biologische Schwierigkeiten, da Pumpen, Schläuche und Anschlußstücke zusätzlich zum Bioreaktor und dem Mittel selbst steigendem Druck und steigender Strömungsgeschwindigkeit ausgesetzt werden.

Ein Bioreaktor vom Hohlfasertyp, welcher diese Probleme berücksichtigt, ist so konstruiert, daß er die Sauerstoffzufuhr zur Zellumgebung unabhängig von der Strömungsrate des Mittels ermöglicht.

Eine Vorrichtung vom Hohlfasertyp, welche eine getrennte Zufuhr von Sauerstoff und Nährstoffen zu den Zellen bewirkt, geht aus der US Patentschrift 5.079 168 hervor, welche auf den Mitanmelder Endotronics, Inc. zurückgeht. Diese Zellkultureinheit weist mindestens eine Hülle auf, die aus einer ersten und einer zweiten porösen Membranschicht zusammengesetzt ist, welche fÜr Gase im hohen Maß durchlässig, jedoch für Zellen und Flüssigkeiten im wesentlichen undurchlässig sind.

Die Hülle ist spiralförmig um einen verlängerten Kern gewikkelt und bildet einen sich zwischen den HÜllen spiralförmig erstreckenden Gasraum. Entsprechende Einrichtungen sind vorgesehen, um Nährstoffe und Zellen in den Zellkulturraum einzubringen und zusätzliche Einrichtungen sind vorhanden, um ein Gas dem Zellkulturraum durch die ersten und zweiten Membranschichten zuzuführen. Ferner sind auch Einrichtungen zur Entsorgung vorgesehen, um flüssige Stoffwechsel-Abfallprodukte und Zellprodukte aus dem Zellkulturraum und gasförmige Abfallprodukte aus dem Zellkulturraum durch die ersten und zweiten Nembranschichten zu entfernen.

Eine weitere Vorrichtung vom Hohlfasertyp, welche die getrennte Zufuhr von Sauerstoff und Nährstoffen zu den Zellen bewirkt, geht aus der US PS 5.416.022, hervor, welche ebenfalls auf den Mitanmelder Endotronics, Inc., zurückgeht. Bei dieser Vorrichtung vom Hohlfasertyp sind erste und zweite Membranschichten entlang der Kanten miteinander versiegelt, um eine Hülle mit dazwischenliegendem Zellkulturraum zu bilden. Die ersten und zweiten Membranschichten sind porös und im hohen Maß für Gase durchlässig aber nahezu undurchlässig für Zellen und Flüssigkeiten. Die Hülle ist zwischen oberen und unteren starren Platten angeordnet, welche ein Gehäuse bilden, um die Hülle zu halten. Eine Vielzahl solcher Hüllen kann innerhalb eines Gehäuses gestapelt werden, wobei das Gehäuse einen zwischen den HÜllen befindlichen Gasraum bildet, welcher der Gaszufuhr zu dem Zellkulturraum durch die poröse Membran dient. Es sind geeignete Einrichtungen vorhanden, um Nährstoffe und Zellen dem Zellkulturraum und Cas dem zwischen den Hüllen befindlichen Gasraum zuzuführen. Ferner sind Einrichtungen zur Entsorgung vorgesehen, um Stoffwechsel-Abfallprodukte und Zellprodukte aus dem Zellkulturraum und gasförmige Abfallprodukte aus dem HÜllen-Zwischenraum zu entfernen.

Aus der EP-A-0 334 506 ist eine Kultur-Vorrichtung bekannt, welche verschiedene Druckverhältnisse im Zellkulturraum und im Gasraum aufführt. Hierbei handelt es sich jedoch um die Erhöhung des Druckes im Gasraum, um die Gaszufuhr zu erleichtern.

Während die Verwendung gasdurchlässiger Membranen, welche in direktem Kontakt mit der Zellumgebung stehen, ein wirksames Verfahren zur Versorgung von Zellen mit Sauerstoff darstellt, müssen die Versorgungsgeräte den Problemen angepaßt werden, die diesem Bioreaktortyp eigen sind. Insbesondere muß der Druck, der auf der Seite der Zellumgebung der gasdurchlässigen Membran anliegt, den Druck auf der Gasraumseite der gasdurchlässigen Membran überschreiten. Wird diese Bedingung nicht aufrechterhalten, treibt das Gas durch die gasdurchlässige Memhran und verdrängt das flüssige Mittel der Zellumgebung, wodurch die Kultur sogar abgetötet werden kann.

Aufgabe der vorliegenden Erfindung ist es daher das Eintreten dieser Bedingung zu verhindern und insbesondere die Verdrängung der flüssigen Medien im Zellkulturraum des Bioreaktors auszuschließen.

Weitere Vorteile ergeben sich durch die Verwendung eines Bioreaktors mit gasdurchlässigen Membranen, welche in direktem Kontakt mit der Zellumgebung stehen, im Gegensatz zur Sauerstoffsättigung von flüssigen, dem Bioreaktor zugeführten Medien. Da keine Notwendigkeit besteht, das flüssige, in den Bioreaktor eintretende Medium mit Sauerstoff anzureichern, sind die Flußraten der Nährstoff zufuhr und der Abfallentfernung gleich groß.

Das System, das in der vorliegenden Anmeldung beschrieben wird, führt dem Bioreaktor flüssiges Medium direkt zu und verringert dadurch den Aufwand und die Kosten der Versorgungsgeräte.

Ein Problem, das sich durch Weglassen der Speichergefäße des Mediums ergibt, bezieht sich auf die abnehmende Gaslöslichkeit des Mediums, wenn dessen Temperatur zunimmt. Um die Lebensdauer des Mediums zu verlängern wird es oft gekühlt. Wenn es in den Inkubator eintritt, erhöht es seine Temperatur und gast aus, wodurch Blasen im Medium erzeugt werden. Normalerweise würden diese in ein zwischengeschaltetes Speichergefäß abgelassen werden, aber die direkte Zufuhrmethode gemäß der vorliegenden Erfindung gestattet nicht das Ablassen der Blasen.

Dadurch könnten die Blasen einen teilweisen oder vollständigen Luftverschluß im Zuführungsweg für das Medium innerhalb des Bioreaktors verursachen. Mit der vorliegenden Erfindung wird das Auftreten dieses Zustandes verhindert.

Die vorliegende Erfindung stellt ein neues und verbessertes System dar, um die relativen Drücke innerhalb eines Bioreaktors vom Hohlfasertyp zu steuern und zu regeln und die Verdrängung des wäßrigen Mediums des Zellkulturraumes durch Gas zu verhindern.

Die vorliegende Erfindung liefert ferner ein neues und verbessertes Drucksteuer- und -regelsystem für einen Bioreaktor, wobei der zellkulturseitige Druck den Druck des umgebenden Gasraumes übersteigt, um den Zellkulturraum vor der Verdrängung durch Luft zu bewahren.

Der Bioreaktor nach der Erfindung hält den Druck des Flüssigraumes für die Zellen in vorteilhafter Weise positiv im Vergleich zum Druck des umgebenden Gasraumes, und zwar mit Hilfe eines auf die Medienschläuche wirkenden positiven Druckmechanismus.

Nach der vorliegenden Erfindung wird ferner ein Mittel vorgeschlagen, um ungelöste Gase aus dem wäßrigen Medium zu entfernen, welches dem Bioreaktor zugeführt wird.

Die Erfindung wird anhand der Zeichnung, in der einige Ausführungsbeispiele dargestellt sind, näher beschrieben. Hierbei zeigen:
- FIGUR 1: ein Diagramm eines negativen Drucksystems für einen Bioreaktor in schematischer Darstellung, gemäß einer AusfÜhrungform der Erfindung;
- FIGUR 2: ein Diagramm eines positiven Drucksystems für einen Bioreaktor in schematischer Darstellung, gemäß einer zweiten AusfÜhrung der Erfindung;
- FIGUR 3: den Querschnitt eines Bioreaktors in schematischer Darstellung mit einem Gaseinlaß und einem Gasauslaß, die von einem Medieneinlaß und einem Medienauslaß getrennt sind;
- FIGUR 4: den Querschnitt eines weiteren Bioreaktors in schematischer Darstellung mit einem Gaseinlaß und einem Gasauslaß, die von einem Medieneinlaß und einem Medienauslaß getrennt sind;
- FIGUR 5: den Querschnitt eines Flußbegrenzers fÜr einen Medienfluß entsprechend der ersten Ausführungform der vorliegenden Erfindung;
- FIGUR 6: den Querschnitt eines Flußbegrenzers für einen Medienfluß entsprechend der zweiten Ausführungform der vorliegenden Erfindung mit einem Ventil ingeschlossener Position.
- FIGUR 7: den Flußbegrenzer nach Figur 6 in offener Ventilstellung;
- FIGUR 8: eine schematische Darstellung eines Bioreaktors im Querschnitt ähnlich der Ausführungsform nach Fig. 3 mit Medieneinlaß, der für den direkten Kontakt mit dem Gasraum modifiziert wurde;
- FIGUR 9: ein Diagramm in schematischer Darstellung für ein System zur Analyse von fünf separaten Zell-Linien mit gleichem Zellkulturmedium; und
- FIGUR 10: ein Diagramm in schematischer Darstellung, ähnlich wie Figur 9, jedoch zur Analyse einer Reihe von ähnlichen Zell-Linien mit verschiedenen Zellkulturmedien.

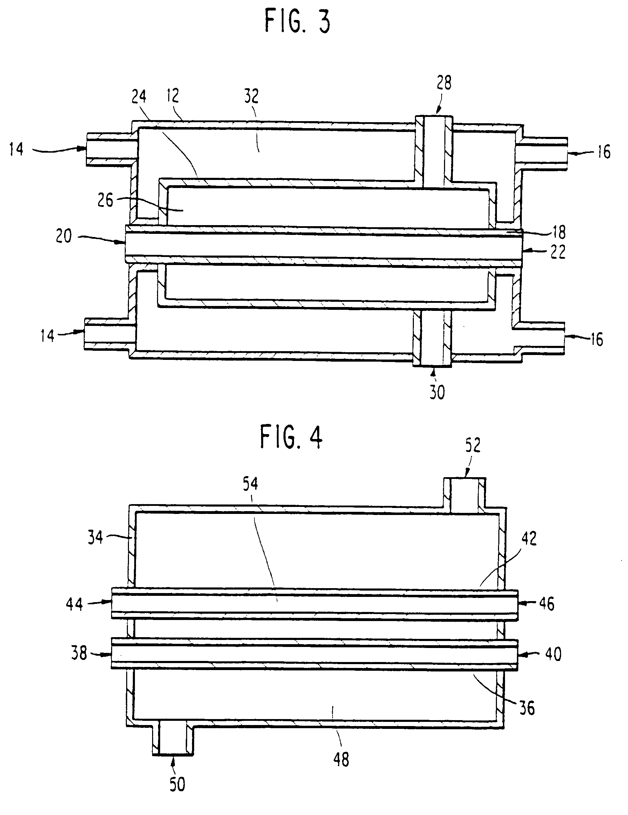

Die Verwendung von gasdurchlässigen Membranen, welche im direkten Kontakt mit der Zellumgebung eines Bioreaktors stehen, stellt eine sehr wirksame Methode zur Sauerstoffversorgung von lebenden Zellen dar. In solchen Bioreaktoren muß jedoch der Zellkulturraum auf einen Druck gehalten werden, der über dem Druck im Gasraum liegt, um den Zellraum davor zu schützen, durch den Gasdruck verdrängt zu werden. Ein solcher Bioreaktor ist schematisch in den Fig. 3 und 4 dargestellt.

Wie aus Figur 3 ersichtlich ist, weist das starre Bioreaktorgehäuse 12 zwei Gaseinlaßanschlüsse 14 und zwei Gasauslaßanschlüsse 16 auf, um dem Bioreaktor Sauerstoff zuführen und gasförmige Abfallprodukte abführen zu können. Mindestens ein hohlrohrartiger Durchgang 18 erstreckt sich durch das Bioreaktorgehäuse 12, wobei der Einlaß 20 zwischen den Gaseinlässen 14 und der Auslaß 22 zwischen den Auslässen 16 angeordnet ist. Eine hydrophobe gasdurchlässige Membran 24 umgibt den hohlrohrartigen Durchgang in einem solchen Abstand, daß ein Zellkulturraum 26 gebildet wird. Der Zellkulturraum 26 ist mit einem Einlaß 28 versehen, um ihm Zellen zuführen zu können und weist einen Auslaß 30 auf, um Zellen und/ oder von den Zellen ausgeschiedene Produkte ernten zu können.

Die Membran 24 besteht aus hydrophobem Material, welches porös und im hohen Maß gasdurchlässig ist, jedoch nahezu undurchlässig für Zellen und Flüssigkeiten. Der hohlrohrförmige Durchgang 18 besteht aus einer hydrophilen Membran, welche semipermeabel ausgebildet ist und den Durchtritt von Nährstoffen vom Nährmedium in den Zellkulturraum 26 erlaubt und die Entfernung von Abfallstoffen vom Zellkulturraum zum Medium zur Entleerung durch Auslaß 22 ermöglicht. Da das hydrophile Material der Membran 18 nicht starr ist und nur eine geringe strukturelle Festigkeit aufweist, ist es notwendig, einen Druck im Zellkulturraum aufrecht zu erhalten. Hierbei muß der Druck im Zellkulturraum 26 höher als der Druck im Gasraum 32 sein, um die Verdrängung des flüssigen Mediums in dem Zellkulturraum des Bioreaktors durch Gas zu verhindern.

Der Bioreaktor, der schematisch in Figur 3 dargestellt ist, ist im Prinzip dem Zellkulturapparat sehr ähnlich, der in der US-PS 5.416.022 dargestellt ist.

Das System gemäß vorliegender Erfindung kann auch mit einem Bioreaktor nach Figur 4 verwendet werden, wobei das im wesentlichen starre Bioreaktorgehäuse 34 mit mindestens einem hohlrohrförmigen Durchlaß 36 aus hydrophobem Material versehen ist, der einen Nährstoff-Einlaß 38 und einen Medienauslaß 40 besitzt. Mindestens ein hohlrohrförmiger Durchlaß, bezeichnet als Gasraum 42, erstreckt sich durch das Gehäuse 34 parallel zum Durchlaß 36. Die Wand des Gasraums 42 besteht aus hydrophobem, gasdurchlässigem Material und ist mit einem Gaseinlaß 44 und einem Gasauslaß 46 versehen. Der gesamte Raum innerhalb des Gehäuses 34, der die röhrenförmigen Teile 36 und den Gasraum 42 umgibt, definiert den Zellkulturraum 48. Der Zellkulturraum ist mit einem Einlaß 50 für die Einführung eines flüssigen Mediums und von Zellen versehen und mit einem Auslaß 52 fÜr die Ernte von Zellen und/oder den von den Zellen ausgeschiedenen Produkten.

Wie in der vorherigen Ausführung zu Figur 3 gezeigt, muß der Druck im Zellkulturraum 48 höher sein als der Druck im Gasraum, um die Verdrängung des flüssigen Mediums im Zellkulturraum durch Gas zu verhindern.

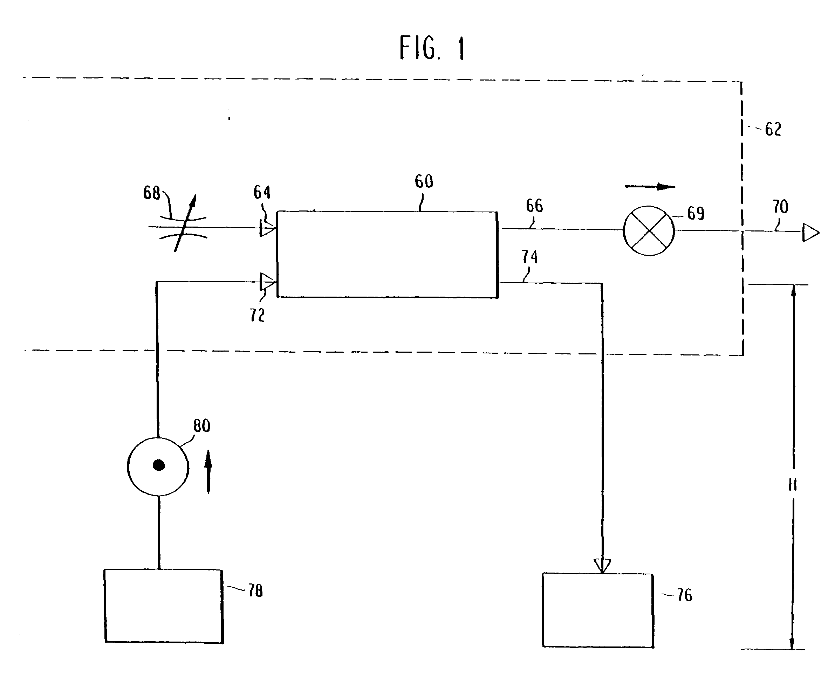

Die relativen Drücke im Zellkulturraum und im Gasraum können im Zusammenwirken mit einem negativen Druckkontrollsystem, wie in Figur 1 gezeigt, reguliert werden. Der Bioreaktor 60 vom in den Figuren 3 und 4 gezeigten Typ oder vom Typ nach der oben genannten US-Patentschrift, ist innerhalb eines Wärmeschranks, einer integrierten Wärmeeinheit mit geregelter Gasversorgung oder eines Inkubators 62 untergebracht, der verschiedene CO₂/ Luft-Mischungen der gewünschten Temperatur enthält. Der Bioreaktor ist mit einem Gaseinlaß 64 und einem Gasauslaß 66 versehen. Der Gasstrombegrenzer 68 ist mit dem Gaseinlaß 64 verbunden und eine Vakuumpumpe 69 ist zwischen dem Gasauslaß 66 und einer Abluftröhre 70 angebracht, die sich vom Inkubator 62 nach außen erstreckt. Der Bioreaktor 60 ist ferner mit dem Einlaß 72 für Flüssigmedium und einem Auslaß 74 versehen, der mit dem Abfallsammelgefäß 76 verbunden ist, welches in einer Entfernung H unter dem Bioreaktor 60 untergebracht ist. Der Nährstoff wird von einer Nährstoffquelle 78 zum Einlaß 72 mittels einer peristaltischen oder Schlauch-Pumpe befördert, welche verschiedene Flußraten erzeugen kann. Bei diesem System ist es wünschenswert, den Druck im Gasraum innerhalb des Bioreaktors unter den Druck im Zellzuchtraum innerhalb des Bioreaktors zu senken. Der Zellraum innerhalb des Bioreaktors befindet sich auf einem negativen Manometerdruck, da das Abfallsammelgefäß sich auf einem niedrigeren Niveau als der Bioreaktor befindet und das Medium mittels eines Syphon-Effektes entfernt wird.

Die Größe des negativen Manometerdruckes im Zellraum ist direkt proportional der vertikalen Distanz H zwischen dem Bioreaktor 60 und dem Abfallsammelgefäß 76.

Der Gasstrombegrenzer 68 in Verbindung mit der Vakuumpumpe 69 hält den Gasraum auf einen Druck, der unter dem von dem Syphoneffekt erzeugten liegt. Typische DrÜcke sind (-60 mm Hg) -80 mbar Syphon und (-120 mm Hg) -160 mbar im Gasraum. Der Gasstrombegrenzer 68 dient dem zusätzlichen Zweck der Steuerung der Gasflußraten, um sicherzustellen, daß Flüssigkeitsverdunstungen Über die hydrophobe Membran innerhalb des Bioreaktors auf einen Wert begrenzt werden, der keine gefährlichen osmoloritätsverhältnisse schafft.

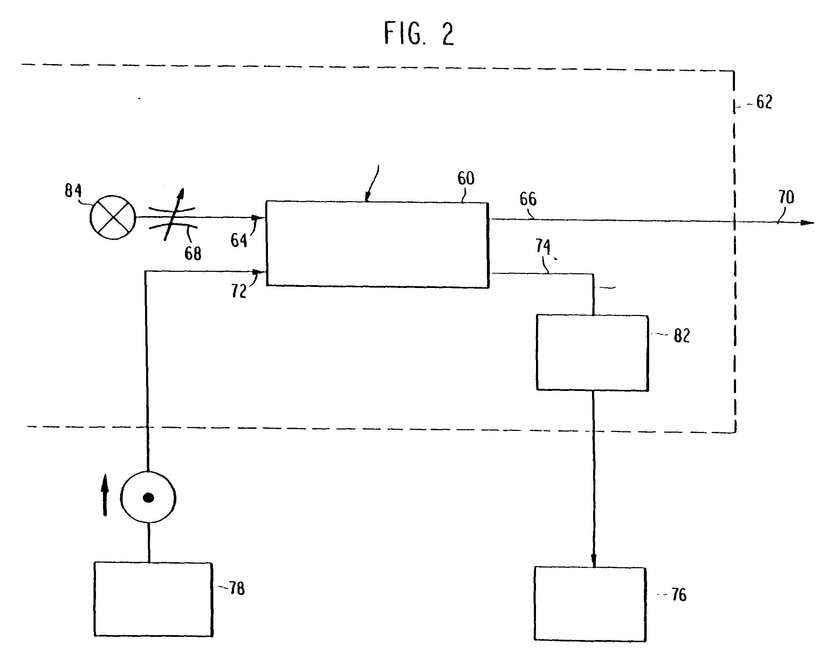

In der in Figur 2 gezeigten Ausführung wird ein positives Drucksystem verwendet, um die Verdrängung des Flüssigmediums im Zellzuchtraum des Bioreaktors durch Gas zu verhindern. Wie in der vorhergehenden Ausführung ist der Bioreaktor 60 innerhalb eines Wärmeschranks, einer integrierten Wärmeeinheit mit geregelter Gasversorgung oder innerhalb eines Inkubators 62 untergebracht und der Bioreaktor ist mit einem Gaseinlaß 64, einem Gasauslaß 66, einem Nährstoffeinlaß 72 und einem Medienauslaß 74 versehen. Nährstoffe werden von einer Nährstoffquelle 78 mittels einer Schlauchpumpe 80 zum Nährstoffeinlaß 72 gefördert. Allerdings ist eine Druckeinheit 82 für positive Drucke zwischen den Medienauslaß 74 und das Abfallsammelgefäß 76 geschaltet. Die Druckeinheit 82 ist dazu bestimmt, den Medienfluß vom Bioreaktor selektiv zu begrenzen, um damit den Druck des Zellzuchtraumes innerhalb des Bioreaktors höher als den Druck des umliegenden Gasraumes zu halten.

In der Ausführung nach Figur 2, fördert die Gaspumpe 84 das Gas zum Einlaß 64 durch einen Gasstrombegrenzer 68, so daß der Druck im Gasraum niedriger gehalten wird als der Druck innerhalb des Zellzuchtraumes.

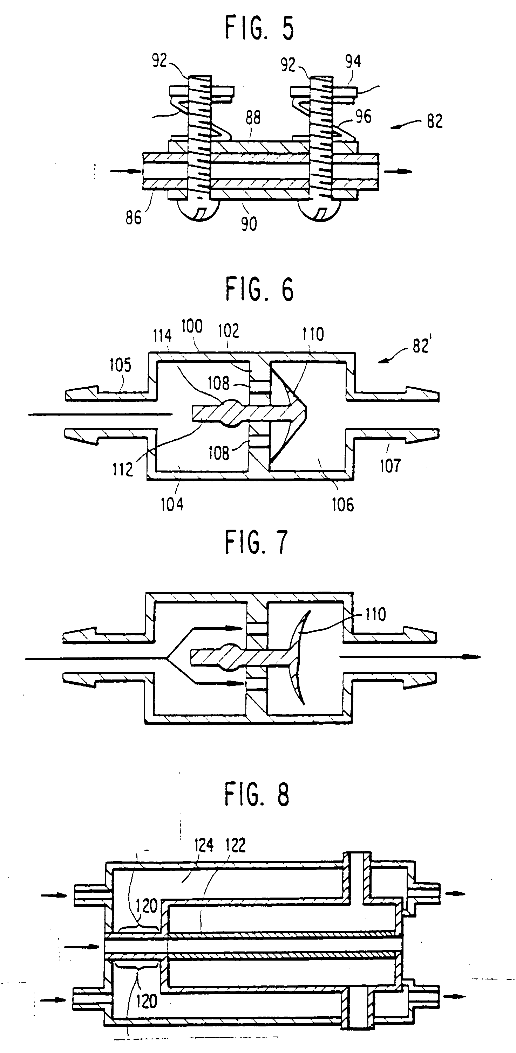

Aus der Figur 5 ist eine geeignete Druckeinheit 82 zur Begrenzung des Medienflusses vom Bioreaktor dargestellt. Der Medienauslaß 74 vom Bioreaktor ist als ein elastischer Schlauch 86 im Bereich der Durchführung durch die positive Druckeinheit 82 ausgeführt. Das elastische Rohr 86 ist zwischen zwei parallelen Platten 88 und 90 angeordnet, welche mehrere Löcher haben, durch die sich Gewindeschrauben 92 erstrecken. Eine Mutter 96 ist auf jedem Bolzen aufgeschraubt und eine Feder 96 ist zwischen jeder Mutter und der Platte 88 montiert. Der Druck, der von den Federn 96 ausgeübt wird, kann durch Drehung der Muttern 94 relativ zu den Schrauben 92 eingestellt werden, um den auf die Röhre 86 wirkenden Druck durch die einander gegenüberstehenden Platten 88 und 90 zu variieren. Je größer der Federdruck ist, um so größer ist die Einengung der Röhre 86, um so den Medienfluß durch die Röhre zu begrenzen.

Eine zweite Ausführung einer positiven Druckeinheit 82' ist in Figur 6 dargestellt. Die Druckeinheit 82' besteht aus einem Ventilkörper 100 mit einer inneren Trennwand 102, welche das Mittelstück des Ventilkörpers in eine Einlaßkammer 104 und eine Auslaßkammer 106 teilt. Die Einlaßkammer 104 ist mit einem EinlaßstÜck 105 versehen, welches zum Anschluß an die vom Bioreaktor kommende Medienauslaßröhre 74 angepaßt ist. Die Auslaßkammer 106 ist mit einem Auslaßdurchgang 107 versehen, welcher zum Anschluß an eine geeignete Röhre vorgesehen ist, die zum Abfallsammelbehälter 76 führt. Die mittlere Teilerwand 102 ist mit einer Vielzahl von Durchgängen 108 ausgestattet und ein flexibler Membranteil 110 Überdeckt die Durchgänge 108 innerhalb der Auslaßkammer 106. Der Ventilstößel 112, der mit dem Ventilteller 110 verbunden ist, erstreckt sich durch die Trennwand 102 und ist mit einer Verbreiterung 114 versehen, um Bewegungen des Ventilstößels zur Rechten zu verhindern, wie in den Figuren 6 und 7 dargestellt ist..

In Figur 6 schließt der Ventilteller 110 den Durchgang 108 wenn der Druck innerhalb des Zellzuchtraumes geringer als ein bestimmter festgelegter Druck ist (z.B. ca. 1 - 3 psi.) 1 - 3 bar. Wenn der Druck den festgelegten Öffnungsdruck überschreitet, (beispielsweise innerhalb des Bereichs von 1-3 bar), bewegt sich der Ventilteller 110 in die "offene"-Position wie in Figur 7 gezeigt, um den Flüssigkeitsstrom durch den Ventilkörper zu ermöglichen.

Auf diese Weise sind beide positiven Druckeinheiten 82 und 82' in der Lage, einen passenden Druck innerhalb des Zellkulturraumes des Bioreaktors aufrechtzuerhalten, welcher größer als der Druck im Gasraum ist. Der Gasstrombegrenzer 68 ist verstellbar, um einen genügend niedrigen positiven Druck innerhalb des Gasraumes zu gewährleisten.

Die Verwendung eines Bioreaktors mit gasdurchlässigen Membranen gewährt einen zusätzlichen Vorteil der Eliminierung von zwischengeschalteten Medien-Speichergefäßen, die den meisten Perfusionstechnologien gemeinsam sind, welche die Hohlfaser oder durchlässige Flachmembranen verwenden. Das in der vorliegenden Erfindung beschriebene System sorgt für die Entfernung ungelösten Gases, welches im flüssigen Medium bei steigenden Temperaturen auftritt. Dies wird dadurch erreicht, daß der Teil 120 der Medienzufuhrröhre 122 in Kontakt mit dem Gasraum 124 gebracht wird, wie in Figur 8 gezeigt ist. Blasen im Medium gelangen durch den Teil 120 des Medienzufuhrrohres in den Gasraum innerhalb des Bioreaktors.

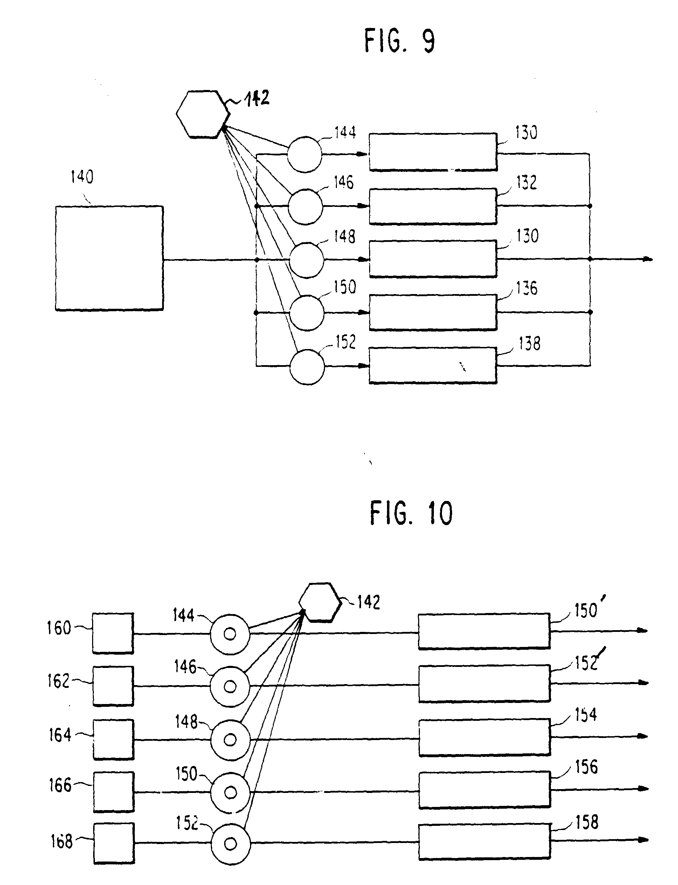

Ein weiterer Vorteil von getrennter Gas- und Medienzufuhr zum Bioreaktor ist in den Figuren 9 und 10 dargestellt. In Figur 9 werden fünf verschiedene Zell-Linien 130, 132, 134, 136 und 138 mit einem Kulturmedium aus einer gemeinsamen Quelle 140 versorgt. Ein einziger Motor 142 dient dazu die individuellen Pumpen 144, 146, 148, 150 und 152 anzutreiben, die jeder Zell-Linie zugeordnet sind. Entsprechend können fünf gleichartige oder ähnliche Zell-Linien 150',152', 154-158 mit fünf verschiedenen Kultur-Medien und fünf verschiedenen Quellen 160-168 analysiert werden. Wiederum wird ein gemeinsamer Motor 142 verwendet, um die fünf einzelnen Pumpen 144-152 anzutreiben, die jeweils zwischen jeder Medienquelle und Zell-Linie geschaltet sind.

## Patentansprüche

1. Drucksteuer- und Druckregelsystem für eine Vorrichtung zur Zellkultivierung und Gewinnung von Zellprodukten (Bioreaktor), mit einem Gasraum (32,42), einem Zellkulturraum (26,48) sowie Einrichtungen zur Gaszufuhr (64) und Gasabfuhr (66), die mit dem Bioreaktor verbunden sind, ferner mit Nährstoffquellen (78) zur Zufuhr von flüssigen Nährstoffen und zur Entfernung flüssiger Abfallprodukte (76), welche getrennt von den Einrichtungen zur Gaszufuhr und Gasabfuhr an dem Bioreaktor angeschlossen sind, **gekennzeichnet durch** einen Gasstrombegrenzer (68) und eine Vakuumpumpe (69) zur Steuerung und Regelung des Gasdruckes im Gasraum (32 bzw 42), welche mit dem Bioreaktor funktionell verbunden sind, um im Zellkulturraum (26 bzw. 48) einen höheren Druck als im Gasraum (32 bzw. 42) aufrecht zu erhalten, wobei
a) der Zellkulturraum (26) vom Gasraum (32) durch eine gasdurchlässige hydrophobe Membran (24) und vom hohlrohrartigen Durchgang (18) durch eine hydrophile Membran getrennt ist, oder
b) der Zellkulturraum (48) vom Gasraum (42) durch eine gasdurchlässige hydrophobe Membran und vom hohlrohrartigen Durchgang (36), welcher einen Nährstoff-Einlaß (38) aufweist, durch eine hydrophobe membran getrennt ist.

2. Drucksteuer- und Druckregelsystem für eine Vorrichtung zur Zellkultivierung und Gewinnung von Zellprodukten (Bioreaktor), mit einem Gasraum (32,42), einem Zellkulturraum (26,48) sowie Einrichtungen zur Gaszufunr (64) und Gasabfuhr (66), die mit dem Bioreaktor verbunden sind, ferner mit Nährstoffquellen (78) zur Zufuhr von flüssigen Nährstoffen und zur Entfernung flüssiger Abfallprodukte (76), weiche getrennt von den Einrichtungen zur Gaszufuhr und Gasabfuhr an dem Bioreaktor angeschlossen sind, **gekennzeichnet durch** einen Gasstrombegrenzer (68) und eine Druckeinheit (82) zur Steuerung und Regelung des Gasdruckes im Zellkulturraum (26 bzw. 48), welche mit dem Bioreaktor funktionell verbunden sind und im Zellkulturraum (26 bzw. 48) einen höheren Druck als im Gasraum (32 bzw. 42) aufrecht erhalten, wobei
a) der Zellkulturraum (26) vom Gasraum (32) durch eine gasdurchlässige hydrophobe Membran (24) und vom hohlrohrartigen Durchgang (18) durch eine hydrophile Membran getrennt ist, oder
b) der Zellkulturraum (48) vom Gasraum (42) durch eine gasdurchlässige hydrophobe Membran und vom hohlrohrartigen Durchgang (36), welcher einen Nährstoff-einlaß (38) aufweist, durch eine hydrophobe Membran getrennt ist.

3. Drucksteuersystem und Druckregelsystem nach Anspruch 1, **dadurch gekennzeichnet, daß** die Einrichtungen zur Gasentfernung eine Vakuumpumpe (69), und die Einrichtungen zur Zufuhr flüssiger Nährstoffe eine Nährstoffquelle (78) und Pumpen aufweisen und die Einrichtungen zur Entfernung flüssiger Abfallstoffe mit Mitteln (76) zur Sammlung flüssiger Stoffe versehen sind, welche unter der Vorrichtung angeordnet sind, derart, daß ein Syphon-Abfluß vorliegt.

4. Drucksteuersystem und Druckregelsystem nach Anspruch 2 oder 3, **dadurch gekennzeichnet, daß** das Mittel zur Gaszufuhr aus einer Gaspumpe (84) und einem Gasstrombegrenzer (68) besteht, welche mit dem Bioreaktor in Serie verbunden sind, und die Einrichtung zur Zufuhr flüssigen Nährmittels aus einer Nährstoffquelle (78) und einer Pumpe besteht, die mit dem Bioreaktor ebenfalls in Serie verbunden sind und die Mittel zur Entfernung des Abfalls eine Druckeinheit (82) aufweisen, die zu einem Abfallsammelgefäß (76) in Reihe liegt.

5. Drucksteuersystem und Druckregelsystem nach Anspruch 4, **dadurch gekennzeichnet, daß** die Mittel zur Begrenzung des Flüssigkeitstromes einen elastischen Schlauch (86) zum Transport des flüssigen Abfalls aufweisen, der mittels verstellbarer Federn (96), welche auf zusamnenpreßbare Klemmplatten, zwischen denen sich der Schlauch 86 befindet, zum Verschließen des Schlauches einwirken, um hierdurch den Fluß selektiv zu begrenzen.

6. Drucksteuersystem und Druckregelsystem nach Anspruch 2 bis 4, **dadurch gekennzeichnet, daß** die Mittel zur Begrenzung des Flüssigkeitsstromes ein flexibles Einwegventil als Druckeinheit (82') aufweisen, welches an ein Auslaßrohr des Bioreaktors angeschlossen ist.

7. Drucksteuersystem und Druckregelsystem nach Anspruch 1 oder einem der voranstehenden Ansprüche, **dadurch gekennzeichnet, daß** die Zufuhreinrichtung für flüssige Nährmittel im Bioreaktor Durchflußeinrichtungen aufweist, welche mit einem Teil (120) im direkten Kontakt mit dem Gasraum (124) stehen, um die Überführung der in dem Medium eingefangenen Blasen in den Gasraum (124) zu ermöglichen, während ein zweiter Teil (122) mit dem Zellkulturraum in Kontakt steht.

## Claims

1. A pressure control and pressure regulation system for a device for cultivating cells and extracting cell products (bio-reactor), having a gas chamber (32, 42), a cell culture chamber (26, 48) as well as devices for delivering gas (64) and discharging gas (66) connected with the bio-reactor, which are also linked with nutrient sources (78) for feeding in liquid nutrients and for removing liquid waste products (76), which are connected to the bio-reactor but separately from the gas delivery and gas discharge devices, characterised by a gas flow limiter (68) and a vacuum pump (69) for controlling and regulating the gas pressure in the gas chamber (32 or 42), which are operatively connected to the bio-reactor so that a higher pressure can be maintained in the cell culture chamber (26 or 48) than in the gas chamber (32 or 42), wherein
a) the cell culture chamber (26) is separated from the gas chamber (32) by means of a hydrophobic membrane permeable to gas (24) and from the hollow tube-type passage (18) by means of a hydrophilic membrane, or
b) the cell culture chamber (48) is separated from the gas chamber (32) by means of a hydrophobic membrane permeable to gas and from the hollow tube-type passage (36), which has a nutrient inlet (38), by means of a hydrophobic membrane.

2. A pressure control and pressure regulation system for a device for cultivating cells and extracting cell products (bio-reactor), having a gas chamber (32, 42), a cell culture chamber (26, 48) as well as devices for delivering gas (64) and discharging gas (66) connected with the bio-reactor, which are also linked with nutrient sources (78) for feeding in liquid nutrients and for removing liquid waste products (76), which are connected to the bio-reactor but separately from the gas delivery and gas discharge devices, characterised by a gas flow limiter (68) and a pressure unit (82) for controlling and regulating the gas pressure in the cell culture chamber (26 or 48), which is operatively connected to the bio-reactor, and a higher pressure level can be maintained in the cell culture chamber (26 or 48) than in the gas chamber (32 or 42), wherein
a) the cell culture chamber (26) is separated from the gas chamber (32) by means of a hydrophobic membrane permeable to gas (24) and from the hollow tube-type passage (18) by means of a hydrophilic membrane, or
b) the cell culture chamber (48) is separated from the gas chamber (42) by means of a hydrophobic membrane permeable to gas and from the hollow tube-type passage (36), which has a nutrient inlet, by means of a hydrophobic membrane.

3. A pressure control system and pressure regulation system as claimed in claim 1, characterised in that the devices for removing gas comprise a vacuum pump (69) and the devices for feeding in liquid nutrients have a nutrient source (78) and pumps whilst the devices for removing liquid waste products are provided with means (76) for collecting liquid substances, which are arranged underneath the device so that a syphon-generated discharge is created.

4. A pressure control system and pressure regulation system as claimed in claim 2 or 3, characterised in that the means for delivering gas consists of a gas pump (84) and a gas flow limiter (68) connected in series with the bio-reactor, and the device for feeding in liquid nutrient consists of a nutrient source (78) and a pump, which are also connected in series with the bio-reactor, and the means for removing the waste has a pressure unit (82), which is arranged in series with a waste collection vessel (76).

5. A pressure control system and pressure regulation system as claimed in claim 4, characterised in that the means for limiting the fluid flow have an elastically resilient hose (86) for carrying the liquid waste, wherein the hose can be closed off by means of adjustable springs (96) operating clamp plates that can be pushed together and between which the hose is located, so that the flow therethrough can be selectively limited.

6. A pressure control system and pressure regulation system as claimed in claims 2 to 4, characterised in that the means for limiting the flow of liquid have a flexible one-way valve as a pressure unit (82') which is connected to an outlet pipe of the bio-reactor.

7. A pressure control and pressure regulation system as claimed in claim 1 or in one of the preceding claims, characterised in that the device for delivering liquid nutrient in the bio-reactor has passage arrangements, one part of which (120) is in direct contact with the gas chamber (124) in order to allow the bubbles trapped in the medium to be transferred into the gas chamber (124), whilst a second part (122) of thereof is in contact with the cell culture chamber.

## Revendications

1. Système de commande et de régulation de la pression pour un dispositif de culture de cellules et de récupération de produits cellulaires (bioréacteur), qui comporte une enceinte à gaz (32, 42), une chambre de culture de cellules (26, 48) ainsi que des dispositifs d'amenée de gaz (64) et d'évacuation de gaz (66) reliés au bioréacteur, et qui comporte en outre des sources de nutriments (78) pour l'apport de nutriments liquides et pour l'évacuation des résidus liquides (76), qui sont raccordés au bioréacteur séparément des dispositifs d'amenée de gaz et d'évacuation de gaz, caractérisé par un limiteur du courant de gaz (68) et une pompe à vide (69) en vue de la commande et de la régulation de la pression du gaz dans l'enceinte à gaz (32 ou 42), qui sont reliés fonctionnellement au bioréacteur pour maintenir dans la chambre de culture de cellules (26 ou 48) une pression supérieure à celle régnant dans l'enceinte à gaz (32 ou 42), dans lequel:
a) la chambre de culture de cellules (26) est séparée de l'enceinte à gaz (32) par une membrane (24) hydrophobe, perméable aux gaz, et du passage (18) en forme de tube creux par une membrane hydrophile, ou
b) la chambre de culture de cellules (48) est séparée de l'enceinte à gaz (42) par une membrane hydrophobe, perméable aux gaz, et du passage (36) en forme de tube creux, qui présente un orifice (38) d'introduction de nutriments, par une membrane hydrophobe.

2. Système de commande et de régulation de la pression pour un dispositif de culture de cellules et de récupération de produits cellulaires (bioréacteur), qui comporte une enceinte à gaz (32, 42), une chambre de culture de cellules (26, 48) ainsi que des dispositifs d'amenée de gaz (64) et d'évacuation de gaz (66) reliés au bioréacteur, et qui comporte en outre des sources de nutriments (78) pour l'apport de nutriments liquides et pour l'évacuation des résidus liquides (76), qui sont raccordés au bioréacteur séparément des dispositifs d'amenée de gaz et d'évacuation de gaz, caractérisé par un limiteur du courant de gaz (68) et par une unité de pression (82) pour la commande et la régulation de la pression du gaz dans la chambre de culture de cellules (26 ou 48), qui sont reliés fonctionnellement au bioréacteur et qui maintiennent dans la chambre de culture de cellules (26 ou 48) une pression plus élevée que dans l'enceinte à gaz (32 ou 42), dans lequel:
a) la chambre de culture de cellules (26) est séparée de l'enceinte à gaz (32) par une membrane (24) hydrophobe, perméable aux gaz, et du passage (18) en forme de tube creux par une membrane hydrophile, ou
b) la chambre de culture de cellules (48) est séparée de l'enceinte à gaz (42) par une membrane hydrophobe, perméable aux gaz, et du passage (36) en forme de tube creux, qui présente un orifice (38) d'introduction de nutriments, par une membrane hydrophobe.

3. Système de commande de pression et de régulation de pression selon la revendication 1, caractérisé en ce que les dispositifs d'évacuation du gaz présentent une pompe à vide (69), et les dispositifs d'amenée de nutriments liquides présentent une source de nutriments (78) et des pompes, et les dispositifs d'élimination des résidus liquides sont dotés de moyens (76) de collecte de matières liquides qui sont disposés en dessous du dispositif de telle sorte que l'on obtienne une évacuation par siphon.

4. Système de commande de pression et de régulation de pression selon les revendications 2 ou 3, caractérisé en ce que le moyen d'amenée de gaz est constitué d'une pompe à gaz (84) et d'un limiteur du courant de gaz (68) qui sont reliés en série au bioréacteur, et le dispositif d'amenée de nutriments liquides est constitué d'une source de nutriments (78) et d'une pompe qui sont également reliés en série au bioréacteur, et les moyens d'élimination des résidus présentent une unité de pression (82) qui est disposée en série par rapport à un récipient (76) de collecte de résidus.

5. Système de commande de pression et de régulation de pression selon la revendication 4, caractérisé en ce que les moyens de limitation du courant de liquide présentent un tuyau flexible élastique (86) pour le transport des résidus liquides, avec des ressorts ajustables (96) qui agissent sur des plaques de pinçage pressées l'une vers l'autre et entre lesquelles se trouve le flexible 86, pour fermer le tuyau flexible et ainsi limiter sélectivement le courant.

6. Système de commande de pression et de régulation de pression selon les revendications 2 à 4, caractérisé en ce que les moyens de limitation du courant de liquide présentent une soupape anti-retour qui sert d'unité de pression (82'), et qui est raccordée à un tube de décharge du bioréacteur.

7. Système de commande de pression et de régulation de pression selon la revendication 1 ou l'une des revendications précédentes, caractérisé en ce que le dispositif d'amenée de nutriments liquides dans le bioréacteur présente des dispositifs de passage du courant qui sont en contact direct avec l'enceinte à gaz (124), par une partie (120), pour permettre le transfert dans l'enceinte à gaz (124) des bulles piégées dans le milieu, une deuxième partie (122) étant en contact avec la chambre de culture de cellules.
